(19) Europäisches Patentamt / European Patent Office / Office européen des brevets

(11) **EP 4 130 362 B1**

(12) **EUROPEAN PATENT SPECIFICATION**

(45) Date of publication and mention
of the grant of the patent:
**27.05.2026  Bulletin 2026/22**

(21) Application number: **21785611.1**

(22) Date of filing: **12.02.2021**

(51) International Patent Classification (IPC):
*D01H 13/22* (2006.01)    *G01N 21/88* (2006.01)
*G01N 21/89* (2006.01)    *G01N 33/36* (2006.01)
*B65H 63/06* (2006.01)

(52) Cooperative Patent Classification (CPC):
**B65H 63/065; D01H 13/22; G01N 21/8851;
G01N 21/8915; G01N 33/365;** B65H 2701/31

(86) International application number:
**PCT/JP2021/005320**

(87) International publication number:
**WO 2021/205745 (14.10.2021 Gazette 2021/41)**

(54) **THREAD MONITORING DEVICE, THREAD MONITORING METHOD, THREAD WINDER, AND THREAD MONITORING SYSTEM**

FADENÜBERWACHUNGSVORRICHTUNG, FADENÜBERWACHUNGSVERFAHREN, FADENWICKLER UND FADENÜBERWACHUNGSSYSTEM

DISPOSITIF DE SURVEILLANCE DE FIL, PROCÉDÉ DE SURVEILLANCE DE FIL, ENROULEUR DE FIL ET SYSTÈME DE SURVEILLANCE DE FIL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.04.2020  JP 2020068487**

(43) Date of publication of application:
**08.02.2023  Bulletin 2023/06**

(73) Proprietor: **Murata Machinery, Ltd.
Kyoto-shi,
Kyoto 601-8326 (JP)**

(72) Inventors:
• **NAKADE Kazuhiko
Kyoto-shi, Kyoto 612-8686 (JP)**
• **MINAMINO Katsushi
Kyoto-shi, Kyoto 612-8686 (JP)**

(74) Representative: **Hoffmann Eitle
Patent- und Rechtsanwälte PartmbB
Arabellastraße 30
81925 München (DE)**

(56) References cited:
WO-A1-2014/054528    JP-A- 2005 299 037
JP-A- 2018 177 380    JP-A- 2019 128 262
JP-A- H02 257 382    JP-A- H11 325 841

• **CARVALHO V ET AL: "A comparison of mass parameters determination using capacitive and optical sensors", PROCEDIA CHEMISTRY, ELSEVIER, AMSTERDAM, NL, vol. 1, no. 1, 1 September 2009 (2009-09-01), pages 766 - 769, XP026799658, ISSN: 1876-6196, [retrieved on 20090901]**

## Description

### Technical Field

The present invention relates to a yarn monitoring device, a yarn monitoring method, a yarn winder, and a yarn monitoring system.

### Background Art

As a conventional yarn monitoring device, for example, a device described in JP 2005-299037 A1 is known according to the preamble of claim 1. The yarn monitoring device described in JP 2005-299037 A1 calculates the deviation of a detection signal corresponding to the thickness of traveling yarn, and checks abnormality of a yarn surface layer on the basis of a resultant value of the calculation.

Further yarn monitoring devices are known from CARVALHO V ET AL: "A comparison of mass parameters determination using capacitive and optical sensors", PROCEDIA CHEMISTRY, ELSEVIER, AMSTERDAM, NL, vol. 1, no. 1, 1 September 2009 (2009-09-01), pages 766-769, XP026799658, ISSN: 1876-6196 and JP H02 257382 A.

### Summary of Invention

### Technical Problem

Examples of yarn defects to be detected by the yarn monitoring device include unevenness in yarn thickness (variations in apparent thickness) and excessive hairiness. Conventional yarn monitoring devices have difficulty in detecting excessive hairiness while distinguishing it from unevenness in yarn thickness.

It is an object of an aspect of the present invention to provide a yarn monitoring device, a yarn monitoring method, a yarn winder, and a yarn monitoring system that can estimate hairiness quantity with high accuracy by a simple process while detecting the presence of abnormality in appearance of yarn.

### Solution to Problem

As a result of diligent study to achieve the above object, the inventors of the present invention have found that a predetermined frequency component of a detection signal that varies with yarn thickness is less likely to be affected by unevenness in thickness of a body part of yarn. The inventors have further found that the hairiness quantity can be estimated with high accuracy by a simple process of extracting a predetermined frequency component from the detection signal, calculating the standard deviation or the mean deviation of the predetermined frequency component, and using this value, resulting in having made the present invention.

A yarn monitoring device according to an aspect of the present invention as defined in claim 1 comprises a light emitter configured to emit light toward a traveling area where yarn travels; a light receiver disposed opposite to the light emitter across the traveling area and configured to receive the light emitted from the light emitter and partially blocked by the yarn and to output a detection signal corresponding to the amount of the light received; and a detection section configured to detect, based on the detection signal, a state of the yarn traveling in the traveling area, in which the detection section estimates hairiness quantity of the yarn by extracting a predetermined frequency component from the detection signal and calculating a standard deviation or a mean deviation of the extracted predetermined frequency component.

In the yarn monitoring device according to this aspect of the present invention, the detection section estimates the hairiness quantity of the yarn by calculating the standard deviation or the mean deviation of the predetermined frequency component. The predetermined frequency component of the detection signal is less likely to be affected by unevenness in thickness of a body part of the yarn. This enables the yarn monitoring device to distinguish between unevenness in thickness of the yarn and excessive hairiness of the yarn and detect them. Thus, the yarn monitoring device can estimate the hairiness quantity with high accuracy by a simple process of extracting the predetermined frequency component and calculating the standard deviation or the mean deviation, without performing complicated processing such as image processing of two-dimensional images captured by a camera. Consequently, the yarn monitoring device can estimate the hairiness quantity with high accuracy by the simple process while detecting the presence of abnormality in the appearance of the yarn.

In an embodiment, the detection section may determine whether the hairiness quantity is excessively large, based on a result of comparison between the standard deviation or the mean deviation and a threshold. In this configuration, the standard deviation or the mean deviation is compared with the threshold, and if the standard deviation or the mean deviation exceeds the threshold, the hairiness quantity can be determined to be excessively large. Thus, whether the hairiness quantity is excessively large can be properly determined.

In an embodiment, the detection section may compare the standard deviation or the mean deviation with a threshold and output a result of this comparison. In this configuration, the standard deviation or the mean deviation is compared with the threshold, and the hairiness quantity can be determined to be large if the standard deviation or the mean deviation exceeds the threshold, and the hairiness quantity can be determined to be small if not exceeding the threshold. Thus, the state of the hairiness can be output by outputting the comparison result. This allows an operator, for example, to check the state of the hairiness.

According to the invention, the detection section extracts the predetermined frequency component by a frequency filter. In this configuration, the predetermined frequency component can be properly extracted.

According to the invention, a time corresponding to a lower frequency limit of a passband in the frequency filter is set between a time for which the yarn travels a length of 1 cm and a time for which the yarn travels a length of 5 cm.

In an embodiment, the detection section may detect presence of abnormality in appearance of the yarn as the state of the yarn, based on the detection signal. In this configuration, the presence of abnormality in the appearance of the yarn can be detected.

In an embodiment, the light receiver may be a photoelectric conversion element including one pixel.

In an embodiment, the yarn monitoring device may include a holder configured to hold the light emitter and the light receiver and having the traveling area formed therein, and at least parts, in walls of the holder facing the traveling area, that hold the light emitter and the light receiver may be black in color. In this configuration, light can be prevented from being reflected at the walls. Thus, in the yarn monitoring device, light reflected at the walls can be prevented from entering the light receiver. The ratio of light partially blocked by the yarn and light reflected by hairs in the total amount of received light can be relatively increased. Thus, the yarn monitoring device can estimate the hairiness quantity with high accuracy.

In an embodiment, the detection section may calculate a hairiness count by substituting the standard deviation or the mean deviation into a conversion formula. In this configuration, the hairiness count can be calculated in addition to the hairiness quantity.

In an embodiment, the detection section may use the conversion formula corresponding to a type of the yarn when calculating the hairiness count. In this configuration, the hairiness count can be properly calculated in accordance with the type of the yarn.

In an embodiment, the detection section may sample the detection signal at every sampling interval length in the yarn. In this configuration, a sampled value can be obtained at every sampling interval length regardless of the speed of the yarn, and thus the hairiness can be accurately detected.

In an embodiment, the sampling interval length may be 0.5 mm or more and 5 mm or less.

In an embodiment, the detection signal may be a voltage signal that varies with the state of the yarn.

A yarn monitoring method according to an aspect of the present invention as defiend in claim 10 is a yarn monitoring method to be performed by a control section in a yarn monitoring device, in which a light emitter emits light toward traveling yarn, and a light receiver receives light emitted from the light emitter and partially blocked by the yarn, and outputs a detection signal corresponding to the amount of the light received to the control section, the method including: an extraction step of extracting a predetermined frequency component from the detection signal; and a hairiness quantity estimation step of estimating hairiness quantity of the yarn by calculating a standard deviation or a mean deviation of the predetermined frequency component extracted at the extraction step.

In the yarn monitoring method according to this aspect of the present invention, at the hairiness quantity estimation step, the hairiness quantity is estimated by calculating the standard deviation or the mean deviation of the predetermined frequency component. The predetermined frequency component of the detection signal is less likely to be affected by unevenness in thickness of a body part of the yarn. This enables the yarn monitoring method to distinguish between unevenness in thickness of the yarn and excessive hairiness of the yarn and detect them. Thus, the yarn monitoring method can estimate the hairiness quantity with high accuracy by a simple process of extracting the predetermined frequency component and calculating the standard deviation or the mean deviation, without performing complicated processing such as image processing of two-dimensional images captured by a camera. Consequently, the yarn monitoring method can estimate the hairiness quantity with high accuracy by the simple process while detecting the presence of abnormality in the appearance of the yarn.

In an embodiment, at the hairiness quantity estimation step, whether the hairiness quantity is excessively large may be determined based on a result of comparison between the standard deviation or the mean deviation with a threshold. In this method, the standard deviation or the mean deviation is compared with the threshold, and if the standard deviation or the mean deviation exceeds the threshold, the hairiness quantity can be determined to be excessively large. Thus, whether the hairiness quantity is excessively large can be properly determined.

In an embodiment, at the hairiness quantity estimation step, the standard deviation or the mean deviation may be compared with a threshold, and a result of this comparison may be output. In this method, the standard deviation or the mean deviation is compared with the threshold, and the hairiness quantity can be determined to be large if the standard deviation or the mean deviation exceeds the threshold, and the hairiness quantity can be determined to be small if not exceeding the threshold. Thus, the state of the hairiness can be output by outputting the comparison result.

According to the invention, at the extraction step, the predetermined frequency component is extracted by a frequency filter. In this method, the predetermined frequency component can be properly extracted.

According to the invention, a time corresponding to a lower frequency limit of a passband in the frequency filter is set between a time for which the yarn travels a length of 1 cm and a time for which the yarn travels a length of 5 cm.

In an embodiment, at the hairiness quantity estimation step, presence of abnormality in appearance of the yarn may be detected as the state of the yarn, based on the detection signal. In this method, the presence of abnormality in the appearance of the yarn can be detected.

A yarn winder according to an aspect of the invention includes: a yarn feeding section capable of feeding yarn; a winding section configured to wind the yarn fed by the yarn feeding section into a package; a yarn joining section configured to perform yarn joining operation of joining the yarn fed by the yarn feeding section and the yarn wound by the winding section; and the above-described yarn monitoring device, the yarn monitoring device being disposed between the yarn feeding section and the winding section.

The yarn winder according to this aspect of the present invention includes the above-described yarn monitoring device. This enables the yarn winder to estimate the hairiness quantity with high accuracy by the simple process while detecting the presence of abnormality in the appearance of the yarn.

In an embodiment, the yarn winder may include a host device configured to set a measurement length for calculating the standard deviation or the mean deviation per predetermined length of the yarn and output measurement length information on the measurement length to the yarn monitoring device. The yarn monitoring device may calculate the standard deviation or the mean deviation based on the measurement length information output from the host device. In this configuration, the standard deviation or the mean deviation can be calculated for each measurement length regardless of the speed of the yarn, and thus the hairiness can be accurately detected.

A yarn monitoring system according to an aspect of the present invention includes: the above-described yarn monitoring device; and a host device capable of communicating data to and from the yarn monitoring device.

The yarn monitoring system according to this aspect of the present invention includes the above-described yarn monitoring device. This enables the yarn monitoring system to estimate the hairiness quantity with high accuracy by the simple process while detecting the presence of abnormality in the appearance of the yarn.

In an embodiment, the host device may transmit measurement length information on the measurement length to the yarn monitoring device for calculating the standard deviation or the mean deviation for each predetermined length of the yarn, and the detection section may calculate the standard deviation or the mean deviation based on the measurement length information received from the host device. In this configuration, the standard deviation or the mean deviation can be calculated for each measurement length regardless of the speed of the yarn, and thus the hairiness can be accurately detected.

In an embodiment, setting of the measurement length may be changeable in the host device. In this configuration, the setting of the measurement length can be changed in accordance with yarn processing conditions such as yarn types of the yarn.

In an embodiment, the host device may transmit threshold information on a threshold to the yarn monitoring device, and the yarn monitoring device may determine whether the hairiness quantity is excessively large, based on the standard deviation or the mean deviation and the threshold information received from the host device. In this configuration, the standard deviation or the mean deviation is compared with the threshold, and if the standard deviation or the mean deviation exceeds the threshold, the hairiness quantity can be determined to be excessively large. Thus, whether the hairiness quantity is excessively large can be properly determined.

In an embodiment, a hairiness count may be settable in the host device, and the host device may convert hairiness count information on the hairiness count into the threshold information and transmit the threshold information after this conversion to the yarn monitoring device. In this configuration, the threshold can be set in accordance with the hairiness count.

In an embodiment, the yarn monitoring device may transmit the standard deviation or the mean deviation to the host device, and the host device may receive the standard deviation or the mean deviation transmitted from the yarn monitoring device and calculate the hairiness count by substituting the received standard deviation or the received mean deviation into a conversion formula. In this configuration, the hairiness count can be calculated in addition to the hairiness quantity.

In an embodiment, the host device may use the conversion formula corresponding to a type of the yarn when calculating the hairiness count. In this configuration, the hairiness count can be properly calculated in accordance with the type of the yarn.

In an embodiment, the yarn monitoring system may include a display section configured to display the hairiness count calculated by the host device. In this configuration, the hairiness count is displayed on the display section, and thus an operator, for example, can check the hairiness count.

In an embodiment, the detection section may acquire a yarn speed and calculate the standard deviation or the mean deviation for each predetermined length of the yarn, and the display section may display a trend in the hairiness count corresponding to the yarn speed. In this configuration, the operator, for example, can check the trend in the hairiness count.

**Advantageous Effects of Invention**

According to an aspect of the present invention, it is possible to estimate the hairiness quantity with high accuracy by the simple process while detecting the presence of abnormality in the appearance of the yarn.

**Brief Description of Drawings**

[FIG. 1] FIG. 1 is a front view of a spinning machine that is a yarn winder according to an embodiment.
[FIG. 2] FIG. 2 is a side view of the spinning machine illustrated in FIG. 1.
[FIG. 3] FIG. 3 is a diagram schematically illustrating a configuration of a yarn monitoring device.
[FIG. 4] FIG. 4 is a graph illustrating a relation between frequency and voltage as a result of analysis of detection signals by an FFT analyzer.
[FIG. 5] FIG. 5 is a front view of an automatic winder that is a yarn winder according to another embodiment.

**Description of Embodiments**

Preferred embodiments will now be described in detail with reference to the attached drawings. In the description of the drawings, like or equivalent elements are designated by like reference signs, and duplicate description is omitted.

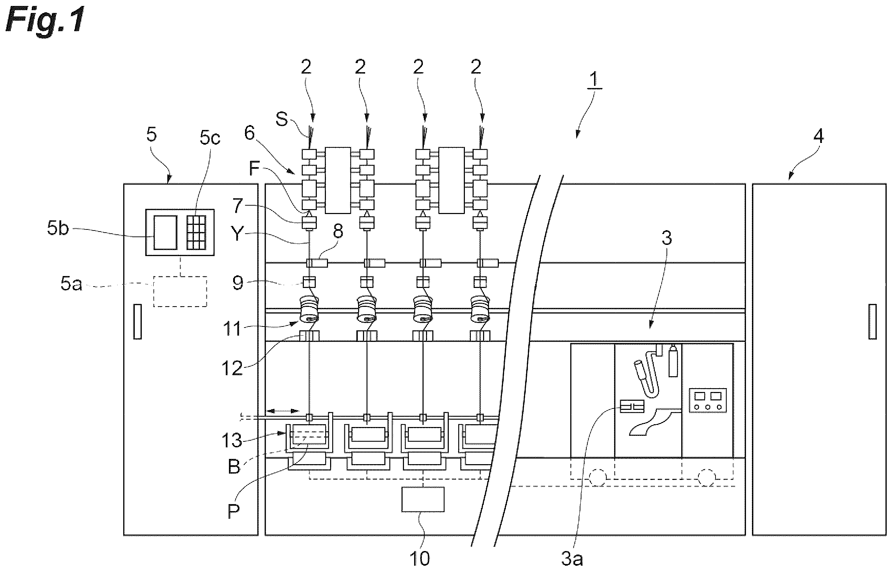

As illustrated in FIG. 1, an air-jet spinning machine (yarn winder) 1 includes a plurality of spinning units (winding units) 2, a yarn joining cart 3, a doffing cart (not illustrated), a first end frame 4, and a second end frame 5. The spinning units 2 are aligned in a row. Each spinning unit 2 forms yarn Y and winds it into a package P. When yarn Y has been cut or the yarn Y has broken for some reason in a certain spinning unit 2, the yarn joining cart 3 performs yarn joining operation in the spinning unit 2. When a package P has been fully wound on a certain spinning unit 2, the doffing cart doffs the package P, and supplies a new bobbin B to the spinning unit 2.

The first end frame 4 accommodates, for example, a collection device configured to collect fiber waste, yarn waste, and the like generated in the spinning unit 2. The second end frame 5 accommodates, for example, an air supply unit configured to adjust air pressure of compressed air (air) to be supplied to the air-jet spinning machine 1 and supply the air to the respective sections of the air-jet spinning machine 1 and a drive motor configured to supply power to the respective sections in the spinning units 2. The second end frame 5 includes a machine control device (detection section) 5a, a display screen (display section) 5b, and input keys 5c. The machine control device 5a centrally manages and controls the respective sections of the air-jet spinning machine 1. The display screen 5b can display, for example, information on settings and/or the status of the spinning units 2. An operator can make the settings of the spinning units 2 by performing appropriate operations with the input keys 5c.

Each spinning unit 2 includes, in the order from the upstream side in a direction in which yarn Y travels, a drafting device 6, an air-jet spinning device (yarn feeding section) 7, a yarn monitoring device 8, a tension sensor 9, a yarn storage device 11, a waxing device 12, and a winding device (winding section) 13. A unit controller 10 is provided for every predetermined number of spinning units 2, and controls operations of the spinning units 2. The unit controller 10 is configured with one or more computer devices, for example. The unit controller 10 includes a central processing unit (CPU), which is a processor, and a random access memory (RAM) or a read only memory (ROM), which is a recording medium. The unit controller 10 executes various controls by loading programs, for example, on hardware such as the CPU and the RAM.

The drafting device 6 drafts a sliver (fiber band) S. The air-jet spinning device 7 forms yarn Y by twisting a fiber band F drafted by the drafting device 6 using swirling airflow.

The yarn monitoring device 8 monitors yarn Y traveling between the air-jet spinning device 7 and the yarn storage device 11 to detect the presence of a yarn defect. Examples of the yarn defect to be detected include excessive hairiness. The examples of the yarn defect may further include at least one of a nep, a slub, a yarn color defect, different yarn count abnormality, weak yarn, yarn physical property change, and the like. When having detected a yarn defect, the yarn monitoring device 8 transmits a yarn defect detection signal to the unit controller 10. The yarn monitoring device 8 and the machine control device 5a can communicate data via the unit controller 10. The yarn monitoring device 8 and the machine control device 5a constitute a yarn monitoring system.

The tension sensor 9 measures the tension of traveling yarn Y between the air-jet spinning device 7 and the yarn storage device 11, and transmits a tension measurement signal to the unit controller 10. When the unit controller 10 has determined that abnormality has occurred based on detection results of the yarn monitoring device 8 and/or the tension sensor 9, the yarn Y is cut in the spinning unit 2.

The yarn storage device 11 eliminates slack in yarn Y between the air-jet spinning device 7 and the winding device 13. The waxing device 12 applies wax to yarn Y between the yarn storage device 11 and the winding device 13.

The winding device 13 winds yarn Y onto a bobbin B to form a package P. The winding device 13 includes a cradle arm 14, a winding drum 15, and a traverse mechanism 16. The cradle arm 14 rotatably supports the bobbin B.

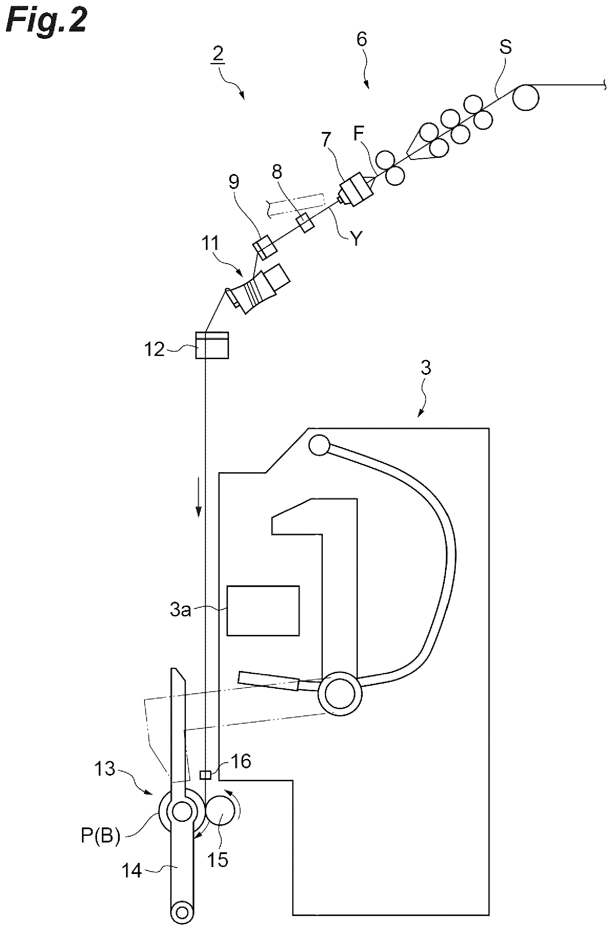

As illustrated in FIG. 2, the yarn joining cart 3 travels to a spinning unit 2 in which yarn Y has been cut, and performs yarn joining operation in this spinning unit 2. The yarn joining cart 3 includes a yarn joining device (yarn joining section) 3a. The yarn joining device 3a performs yarn joining operation of joining yarn Y fed by the air-jet spinning device 7 and yarn Y wound by the winding device 13. The yarn joining device 3a is a splicer using compressed air, or a knotter configured to mechanically join the yarns Y.

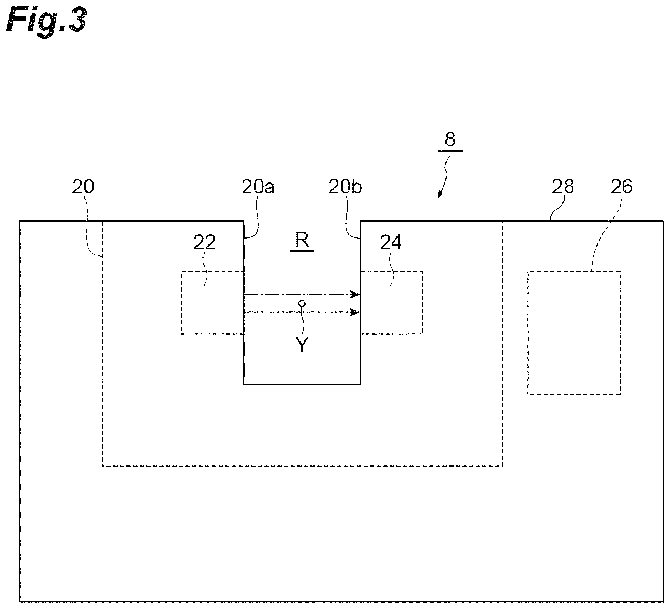

The following describes the yarn monitoring device 8 in detail. As illustrated in FIG. 3, the yarn monitoring device 8 includes a holder 20, a light emitter 22, a light receiver 24, a control section (detection section) 26, and a casing 28. FIG. 3 illustrates a configuration of the yarn monitoring device 8 when viewed from a direction in which yarn Y travels.

The holder 20 holds the light emitter 22 and the light receiver 24. The casing 28 accommodates the holder 20 and the control section 26. The holder 20 has a cubic shape, for example. In the holder 20, a traveling area R is formed. The traveling area R is a space extending along the traveling direction of the yarn Y and being open to the front side of the machine. In other words, the traveling area R is a path through which the traveling yarn Y passes, and is formed in the shape of a groove extending from upstream to downstream in the traveling direction of the yarn Y and being open to the front side of the machine. The traveling area R is defined by walls 20a, 20b of the holder 20. The walls 20a, 20b are black. The light emitter 22 and the light receiver 24 are preferably disposed at the same position in the traveling direction of the yarn Y. More specifically, at least a wall surface of the wall 20a at a position (range), in the traveling direction of the yarn Y, where the light emitter 22 and the light receiver 24 are held is preferably black in color. Herein, the "black" is not limited to strictly pitch black. Because the purpose is to clearly distinguish it from the color of yarn Y (generally white), it only needs to be a dark color that achieves this purpose and may have some saturation.

The light emitter 22 is disposed so as to emit light toward the yarn Y traveling in the traveling area R of the holder 20. The light emitter 22 is disposed on the side of one wall 20a of the pair of walls 20a, 20b that are opposed to each other. For example, a light emitting diode (LED) can be used as the light emitter 22. The luminescent color of the light emitter 22 is not limited to a particular one, and may be blue, yellow, or green, for example. The light emitter 22 may also emit infrared light. The light emitter 22 is driven by a drive circuit (not illustrated).

The light receiver 24 receives light emitted from the light emitter 22. The light receiver 24 is disposed on the side of the other wall 20b of the pair of walls 20a, 20b that are opposed to each other. The light receiver 24 is disposed opposite to the light emitter 22. In other words, the light receiver 24 is disposed, in the holder 20, opposite to the light emitter 22 with the traveling area R therebetween. The light receiver 24 is a photoelectric conversion element including one pixel. For example, a photodiode can be used as the light receiver 24. The light receiver 24 outputs, to the control section 26, a detection signal (analog signal: waveform data) corresponding to the amount of received light that has been partially blocked by the yarn Y. The detection signal is a voltage signal that varies with the thickness (state) of yarn Y.

The control section 26 controls the yarn monitoring device 8. The control section 26 includes a central processing unit (CPU) and a random access memory (RAM) or a read only memory (ROM), which is a recording medium. The control section 26 includes an amplifier, a low-pass filter, and a sample-and-hold circuit (all not illustrated). The control section 26 processes the detection signal output from the light receiver 24 by using the amplifier, the low-pass filter, and the sample-and-hold circuit.

The control section 26 detects the presence of a yarn defect on the basis of the detection signal of the light receiver 24. In the present embodiment, a method for detecting excessive hairiness (excessively large hairiness quantity) as a yarn defect (yarn monitoring method) will be described in detail. The control section 26 extracts a predetermined high-frequency component from the detection signal and detects the hairiness of yarn Y on the basis of the deviation of the high-frequency component. The predetermined high-frequency component means a frequency component higher than a predetermined frequency or a frequency component in a range higher than the predetermined frequency. The predetermined high-frequency component is set in accordance with the type of yarn Y and the speed of yarn Y, for example.

The control section 26 digitally processes the detection signal output from the light receiver 24. The control section 26 performs sampling processing on the analog signal that is the detection signal and converts it into a digital signal. The control section 26 samples the analog signal at a predetermined sampling frequency. The predetermined sampling frequency is set by Equation (1) given below. In the present embodiment, the control section 26 samples the analog signal at every sampling interval length in yarn Y. The sampling interval length is 0.5 mm or more and 5 mm or less. The control section 26 changes the sampling frequency in accordance with changes in yarn speed, and performs the sampling at every fixed length of yarn Y (sampling interval length). In the present embodiment, this fixed length of yarn Y is 1 mm, for example. In other words, the control section 26 performs the sampling at every length of 1 mm of yarn Y, regardless of the yarn speed. The control section 26 may sample the analog signal at regular time intervals regardless of whether the yarn speed changes.

[Equation 1]

```
sampling frequency = yarn speed/sampling interval length
```

The control section 26 analyzes digital signals thus digitally processed. The control section 26 extracts a predetermined frequency component from each digital signal (extraction step). The predetermined frequency component is a frequency band that is not affected by unevenness in thickness of a body portion of yarn Y (variations in apparent thickness). The lower frequency limit of the predetermined frequency component is set based on a period. The period is a time determined based on the traveling speed and the length of yarn Y. The time corresponding to the lower frequency limit of the predetermined frequency component is set between a time for which the yarn Y travels a length of 1 cm and a time for which the yarn Y travels a length of 5 cm. In the present embodiment, the control section 26 extracts, as the predetermined frequency component, a frequency band at or higher than a lower frequency limit corresponding to a time for which the yarn Y travels a length of 2 cm. The predetermined frequency component is a frequency equal to or higher than 250 Hz, for example. For example, the time for which the yarn Y travels a length of 2 cm is 4 ms when the traveling speed of the yarn Y is 5 m/s. In this case, the predetermined frequency component is 250 Hz or higher.

The control section 26 extracts the predetermined frequency component by processing the detection signal with a high-pass filter (frequency filter). The control section 26 extracts the predetermined frequency component by using the high-pass filter with a passband the lower frequency limit of which is 250 Hz. As described above, the cutoff frequency of the high-pass filter varies depending on the yarn speed. Thus, sampling at a fixed period requires a high-pass filter corresponding to the period. However, when sampling is performed at every fixed length of yarn Y as in the present embodiment, the filter coefficient can be set constant.

The control section 26 calculates the standard deviation of extracted high-frequency component signals (hairiness quantity estimation step). The standard deviation is calculated based on a measurement length (e.g., 1 m or more and 20 m or less). The control section 26 calculates the standard deviation for each measurement length of yarn Y (e.g., 10 m). The measurement length is preset in the machine control device (host device) 5a. The machine control device 5a can change the setting of the measurement length. The machine control device 5a outputs (transmits) measurement length information on the measurement length to the yarn monitoring device 8. When having received the measurement length information, the control section 26 calculates the standard deviation based on the measurement length information. The control section 26 may calculate the mean deviation instead of the standard deviation. The mean deviation is also calculated for each measurement length of yarn Y.

When having calculated the standard deviation, the control section 26 compares the value of the standard deviation with a threshold to detect an excessively hairy part. In other words, the control section 26 compares the value of the standard deviation with the threshold to determine whether the hairiness quantity is excessively large. The threshold is a preset value and is stored in a recording medium. The threshold is a value that serves as a criterion for detecting hairiness (excessively large hairiness quantity), and can be changed as needed. The threshold is set based on threshold information transmitted from the machine control device 5a. The machine control device 5a can set the hairiness count of yarn Y. The machine control device 5a converts hairiness count information on the set hairiness count into threshold information, and transmits the threshold information to the yarn monitoring device 8 via the unit controller 10. The yarn monitoring device 8 detects an excessively hairy part, using the threshold based on the received threshold information.

If the value of the standard deviation exceeds the threshold, the control section 26 outputs a control signal (yarn defect signal) to the unit controller 10. In the present embodiment, if the number of times the threshold was exceeded is consecutively equal to or greater than a predetermined number of times, the control section 26 outputs the control signal to the unit controller 10. When having received the control signal, the unit controller 10 causes each spinning unit 2 to perform predetermined processes of issuing an alarm, stopping feeding yarn, and cutting yarn, for example.

The control section 26 outputs the standard deviation to the unit controller 10. When having received the standard deviation, the unit controller 10 outputs it to the machine control device 5a.

The machine control device 5a calculates the hairiness count (the hairiness count per predetermined length). The machine control device 5a calculates a first hairiness count and/or a second hairiness count. The first hairiness count is the number of hairs in a first length range. The first length range is 1 mm or more and less than 3 mm. The second hairiness count is the number of hairs in a second length range that is longer than the first length range. The second length range is 3 mm or more. The length referred to in the first length range and the second length range is the length of a hair protruding radially from the body part of yarn Y. The machine control device 5a calculates the hairiness count by substituting the value of the standard deviation to a predetermined conversion formula. The machine control device 5a uses conversion formulas corresponding to the type of yarn Y when calculating the hairiness count. The conversion formulas depending on the type of yarn Y are stored in the recording medium. The conversion formulas are preset individually for the first hairiness count and the second hairiness count as follows.

First hairiness count = Standard deviation $\times$ a1 + b1
Second hairiness count = Standard deviation $\times$ a2 + b2

In the above formulas, a1 and a2 are coefficients (slopes). In the present embodiment, for example, a1 is greater than a2 (a1 > a2). b1 and b2 are intercepts. In the present embodiment, for example, b1 is greater than b2 (b1 > b2). The machine control device 5a can simultaneously calculate the first hairiness count and the second hairiness count by substituting the standard deviation to each of the above conversion formulas. Each conversion formula is set such that the value is converted into a value corresponding to the measurement value of hairiness measured with a typical existing hairiness measuring instrument (G567 manufactured by Zweigle).

As described above, in the yarn monitoring device 8 of the air-jet spinning machine 1 according to the present embodiment, the control section 26 extracts the predetermined frequency component from the detection signal, and detects hairiness of yarn Y based on the standard deviation of the predetermined frequency component. The machine control device 5a calculates the hairiness count by substituting the standard deviation received from the control section 26 into each conversion formula.

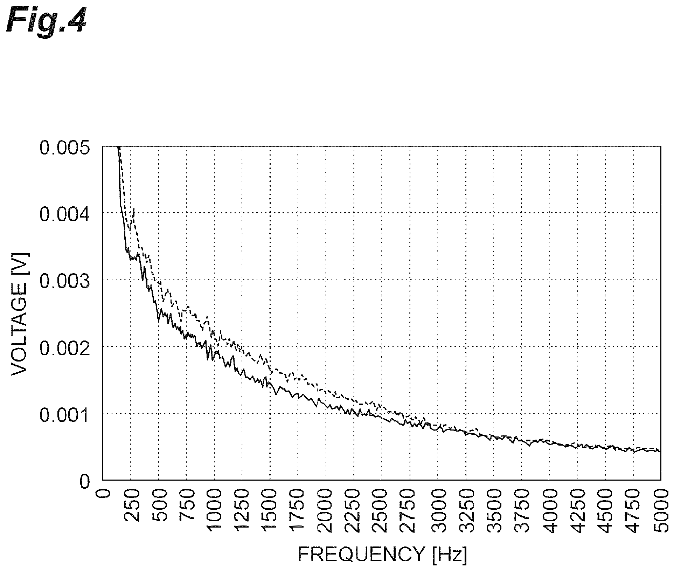

FIG. 4 is a graph illustrating results of analysis of detection signals by the FFT analyzer and a relation between frequency and signal strength. In FIG. 4, the horizontal axis represents frequency [Hz] and the vertical axis represents voltage [V] as signal strength. The plot indicated by the solid line in FIG. 4 represents the result of analysis of yarn Y spun at the standard spinning speed. The plot indicated by the dashed line in FIG. 4 represents the result of analysis of yarn Y spun at a faster speed than the standard spinning speed. The yarn Y spun at a speed faster than the standard spinning speed has more hairiness than the yarn Y spun at the standard spinning speed. In the example in FIG. 4, a significant difference in signal intensity can be observed between the different spinning speeds in the range of about 250 Hz or more and about 2500 Hz or less.

As illustrated in FIG. 4, the two types of yarns the hairiness quantities of which are different exhibit differences in the analysis results in a predetermined frequency band (about 250 Hz or more). Thus, the inventors of the present invention presumed that there is a high correlation between the detection signal and the hairiness quantity in the predetermined frequency band, verified the correlation, and consequently could confirm that results indicating the high correlation could be obtained. It is presumed that the predetermined frequency component of the detection signal is less likely to be affected by unevenness in thickness of the body part of yarn Y. Thus, by detecting hairiness based on the standard deviation of the frequency component of the lower frequency limit corresponding to the frequency band at or higher than a predetermined value, e.g., the time to travel a length of 2 cm, the yarn monitoring device 8 can distinguish between unevenness in thickness of the yarn Y and hairiness (excessively hairy part) of the yarn Y and detect them. Thus, the yarn monitoring device 8 can estimate the hairiness quantity with high accuracy by a simple process of extracting the predetermined frequency component and calculating the standard deviation, without performing complicated processing such as image processing of two-dimensional images captured by a camera. Consequently, the yarn monitoring device 8 can estimate the hairiness quantity with high accuracy by the simple process while detecting the presence of abnormality in the appearance of the yarn Y. Herein, the abnormality in the appearance is, for example, abnormality in thickness (unacceptable unevenness in thickness) of the yarn Y, and does not include abnormality in hairiness quantity (excessive hairiness, etc.).

In the air-jet spinning machine 1 according to the present embodiment, the control section 26 of the yarn monitoring device 8 determines whether the hairiness quantity is excessively large based on a result of comparison between the standard deviation and the threshold. In this configuration, the standard deviation is compared with the threshold, and if the standard deviation exceeds the threshold, the hairiness quantity can be determined to be excessively large. Thus, whether the hairiness quantity is excessively large can be properly determined.

In the air-jet spinning machine 1, the control section 26 of the yarn monitoring device 8 extracts the predetermined frequency component by processing the detection signal with the high-pass filter. In this configuration, the predetermined frequency component can be properly extracted.

In the air-jet spinning machine 1, the yarn monitoring device 8 includes the holder 20 configured to hold the light emitter 22 and the light receiver 24 and having the traveling area R formed therein. In the holder 20, the walls 20a, 20b that hold the light emitter 22 and the light receiver 24 are black in color. In this configuration, light can be prevented from being reflected at the walls 20a, 20b. Thus, in the yarn monitoring device 8, light reflected at the walls 20a, 20b can be prevented from entering the light receiver 24. The ratio of light partially blocked by the yarn Y and light reflected by hairs in the total amount of received light can be relatively increased. Thus, in the yarn monitoring device 8, the accuracy of hairiness detection can be further improved.

In the air-jet spinning machine 1 according to the present embodiment, the machine control device 5a uses the conversion formula corresponding to the type of yarn Y, and outputs the value of a hairiness count calculated by substituting the standard deviation into the conversion formula. In this configuration, in addition to detecting hairiness, the hairiness count can be displayed. The yarn monitoring device 8 can estimate the hairiness quantity while winding the yarn Y. Because the air-jet spinning machine 1 uses the conversion formula to calculate the value of the hairiness count in the machine control device 5a, the conversion formula can be set and managed, for example, centrally in the machine control device 5a. This eliminates the need to set the conversion formula in each of the yarn monitoring devices 8 or to output data relating to the conversion formula to the yarn monitoring devices 8. Thus, the conversion formula can be easily changed, for example.

In the air-jet spinning machine 1 according to the present embodiment, the control section 26 of the yarn monitoring device 8 samples the detection signal at every sampling interval length in yarn Y. The sampling interval length is 1 mm. In this configuration, a sampled value for each fixed yarn length can be obtained regardless of the yarn Y speed, and thus the hairiness can be accurately detected. Furthermore, the filter processing does not have to be changed in accordance with the yarn speed, and thus extraction of the predetermined frequency component can be simplified.

The air-jet spinning machine 1 according to the present embodiment includes the machine control device 5a configured to set the measurement length for calculating the standard deviation per predetermined length of yarn Y and output measurement length information on the measurement length to the yarn monitoring device 8. The yarn monitoring device 8 calculates the standard deviation based on the measurement length information output from the machine control device 5a. In this configuration, the standard deviation for each measurement length can be calculated regardless of the speed of yarn Y, and thus the hairiness can be accurately detected. In the air-jet spinning machine 1, the measurement length is set in the machine control device 5a, and the measurement length information is output from the machine control device 5a to each of the yarn monitoring devices 8 (spinning units 2), and thus the measurement length can be set and managed, for example, collectively in a single machine control device 5a. Thus, the measurement length can be easily changed, for example.

The control section 26 of the yarn monitoring device 8 according to the present embodiment performs sampling processing on the analog signal that is a detection signal and converts it into a digital signal. The control section 26 samples the analog signal at a predetermined sampling frequency. The control section 26 performs filter processing (extraction of the predetermined range frequency) on the digital signal. In this configuration, the frequency range to be extracted (cutoff frequency) can be easily changed. For example, the frequency range to be extracted can be changed in accordance with various yarn processing conditions such as yarn types of yarn Y.

Although the embodiment of the present invention has been described above, the present invention is not necessarily limited to the embodiment described above, and various modifications may be made without departing from the scope of the invention as claimed.

In the above embodiment, an example of a configuration has been described in which the control section 26 extracts the predetermined frequency component by processing the detection signal with the high-pass filter. However, the method of extracting the predetermined frequency component is not limited to this. For example, a bandpass filter (frequency filter) may be used to extract the predetermined frequency component.

In the above embodiment, an example of a configuration has been described in which the machine control device 5a calculates the hairiness count. However, the yarn monitoring device 8 may calculate the hairiness count, or the unit controller 10 may calculate the hairiness count. By sharing the processing among the respective devices, the processing load at each device and/or the communication load between the respective devices can be reduced.

In the above embodiment, an example of a configuration has been described in which the machine control device 5a calculates the first hairiness count and the second hairiness count. However, the machine control device 5a may calculate one type of hairiness count or three or more types of hairiness counts. For example, the machine control device 5a may calculate the hairiness count in the length ranges of "less than 2 mm, less than 3 mm, and 3 mm or more". The conversion formulas can be used to calculate various patterns of hairiness counts by changing the coefficients and the intercepts.

In the above embodiment, an example of a configuration has been described in which the control section 26 outputs the standard deviation to the unit controller 10. However, the control section 26 may compare the standard deviation or the mean deviation with the threshold and output a result of the comparison. In this configuration, the standard deviation or the mean deviation is compared with the threshold, and the hairiness quantity can be determined to be large if the standard deviation or the mean deviation exceeds the threshold, and the hairiness quantity can be determined to be small if not exceeding the threshold. When using two or more thresholds, the control section 26 can classify the state of hairiness into three or more levels. The control section 26 can report (display) the state of hairiness by outputting this comparison result (classification result). In this configuration, the state of hairiness of yarn Y can be reported in real time while the yarn is traveling (during yarn winding). This allows an operator, for example, to check the state of the hairiness.

In the above embodiment, when the standard deviation calculated by the control section 26 satisfies a predetermined condition, the unit controller 10 causes the corresponding spinning unit 2 to perform the predetermined processes of issuing an alarm, stopping feeding yarn, and cutting yarn, for example. However, the air-jet spinning machine 1 does not have to do such predetermined processes. For example, the standard deviation calculated by the control section 26 may be converted into a hairiness count, and the hairiness count may be simply displayed. The standard deviation corresponds to the hairiness quantity. Based on the calculated standard deviation, the air-jet spinning machine 1 can take various actions (display, count, alarm, etc.) relating to the hairiness, and only needs to take at least one of these actions.

In the above embodiment, each spinning unit 2 preferably includes means for detecting (acquiring) the yarn speed. Furthermore, the corresponding yarn monitoring device 8 preferably includes means for detecting the yarn speed. In this case, the control section 26 of the yarn monitoring device 8 can perform sampling corresponding to the sampling interval length based on the detected yarn speed. Herein, the spinning unit 2 may include means for detecting the yarn speed separately from the yarn monitoring device 8. The control section 26 may also perform sampling corresponding to the sampling interval length based on the yarn speed (the yarn speed that is a target winding speed input by the operator) set in a setting device such as the machine control device 5a.

In the above embodiment, the hairiness count can be displayed. However, not only the hairiness count, but also a trend in the hairiness count (or the hairiness quantity) corresponding to the yarn speed may be displayed, for example. In other words, changes in the hairiness count (or the hairiness quantity) may be displayed in response to changes in the yarn speed.

In the spinning unit 2, the yarn storage device 11 has a function of pulling out yarn Y from the air-jet spinning device **7.** However, the yarn Y may be pulled out from the air-jet spinning device 7 by a delivery roller and a nip roller. When the yarn Y is pulled out from the air-jet spinning device 7 by a delivery roller and a nip roller, instead of the yarn storage device 11, a slack tube or a mechanical compensator, for example, configured to absorb slack of the yarn Y using suction airflow may be provided.

In the air-jet spinning machine 1, the respective devices are disposed such that yarn Y fed from the upper side in the height direction is wound at the lower side. However, the respective devices may be disposed such that yarn fed from the lower side is wound at the upper side.

In the air-jet spinning machine 1, at least one of bottom rollers of each drafting device 6 and the corresponding traverse mechanism 16 are driven by power from the second end frame 5 (i.e., driven centrally for a plurality of spinning units 2). However, the respective sections (e.g., the drafting device, the air-jet spinning device, the winding device) of each spinning unit 2 may be driven independently in each spinning unit 2.

The tension sensor 9 may be disposed upstream of the yarn monitoring device 8 in the traveling direction of yarn Y. The unit controller 10 may be provided to each spinning unit 2. In each spinning unit 2, the waxing device 12 and the tension sensor 9 may be omitted.

In FIG. 1, the air-jet spinning machine 1 is illustrated such that cheese-shaped packages P are wound. However, cone-shaped packages P may also be wound. In the case of cone-shaped packages, slack of yarn is generated by traversing the yarn, but this slack can be absorbed by the yarn storage device 11. The materials and shapes of the respective components are not limited to those described above, and various types of materials and shapes may be used.

In the above embodiment, an example of a configuration has been described in which the yarn monitoring device 8 is provided to the air-jet spinning machine 1. However, the yarn monitoring devices may be provided to an automatic winder or a ring spinning machine, for example.

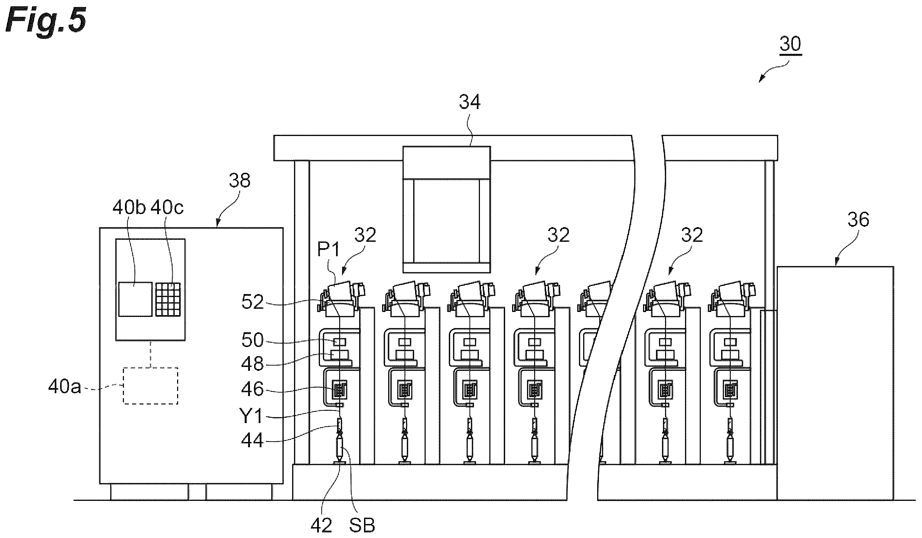

As an example, a configuration in which a yarn monitoring device is provided to an automatic winder will be described. As illustrated in FIG. 5, an automatic winder (yarn winder) 30 includes a plurality of winding units 32, a doffing cart 34, a first end frame 36, and a second end frame 38.

The second end frame 5 includes a machine control device (detection section) 40a, a display screen (display section) 40b, and input keys 40c. The machine control device 40a centrally manages and controls the respective sections of the automatic winder 30.

The winding units 32 are aligned in a row. Each winding unit 32 winds yarn Y1 unwound from a yarn feeding bobbin SB while traversing it, thereby forming a package P1.

The winding unit 32 includes a yarn feeding section 42, a yarn-unwinding assisting device 44, a tension applying device 46, a yarn joining device 48, a yarn monitoring device 50, and a winding device (winding section) 52.

The yarn feeding section 42 holds a yarn feeding bobbin SB, which has been conveyed by a bobbin conveying system (not illustrated), at a predetermined position. The yarn-unwinding assisting device 44 controls a balloon formed by yarn Y1 unwound from the yarn feeding bobbin SB to an appropriate size, thereby assisting smooth unwinding of the yarn Y1. The tension applying device 46 applies a predetermined tension to the traveling yarn Y1. When the yarn monitoring device 50 has detected a yarn defect and cuts the yarn, or when the yarn being unwound from the yarn feeding bobbin SB breaks, for example, the yarn joining device 48 joins lower yarn from the yarn feeding bobbin SB and upper yarn from the package P1. The winding device 52 winds the yarn Y1 onto the package P1 while traversing it.

The yarn monitoring device 50 has the same configuration as that of the yarn monitoring device 8 of the air-jet spinning machine 1. More specifically, the yarn monitoring device 50 includes constituents corresponding to the holder 20, the light emitter 22, the light receiver 24, the control section 26, and the casing 28 of the yarn monitoring device 8.

The yarn monitoring device 50 extracts a predetermined frequency component from a detection signal, and detects the hairiness of yarn Y based on the standard deviation (mean deviation) of the predetermined frequency component. The yarn monitoring device 50 calculates the standard deviation in the extracted high-frequency component signal. The standard deviation is calculated based on a measurement length (e.g., 1 m or more and 20 m or less). The yarn monitoring device 50 calculates the standard deviation per predetermined length (e.g., per 10 m) of yarn Y based on the measurement length. The measurement length is preset in the machine control device (host device) 40a. The machine control device 40a outputs (transmits) measurement length information on the measurement length to the yarn monitoring device 50. When having received the measurement length information, the yarn monitoring device 50 calculates the standard deviation based on the measurement length information. The yarn monitoring device 50 may calculate the mean deviation instead of the standard deviation. The mean deviation is also calculated per predetermined length of yarn Y.

When having calculated the standard deviation (mean deviation), the yarn monitoring device 50 compares the value of the standard deviation (mean deviation) with a threshold to detect an excessively hairy part. If the value of the standard deviation (mean deviation) exceeds the threshold, the yarn monitoring device 50 outputs a control signal (yarn defect signal) to the unit controller 10. When having received the control signal, the unit controller 10 causes each winding unit 32 to perform predetermined processes of stopping an alarm and cutting yarn, for example. The yarn monitoring device 50 also outputs the standard deviation to the unit controller 10. When having received the standard deviation, the unit controller 10 outputs it to the machine control device 40a. The machine control device 40a calculates the hairiness count (the hairiness count per predetermined length). The machine control device 40a calculates the first hairiness count and/or the second hairiness count. The machine control device 40a calculates the hairiness count by substituting the value of the standard deviation to the corresponding predetermined conversion formula. The conversion formula is set in accordance with the type of yarn Y. In the case of ring yarn, for example, examples of the type of yarn Y include carded yarn and combed yarn.

As described above, in the automatic winder 30, the yarn monitoring device 50 estimates the hairiness quantity of yarn Y1 based on the standard deviation of the high frequency component. The predetermined high-frequency component of the detection signal is less likely to be affected by unevenness in thickness of the body portion of yarn Y1. This enables the yarn monitoring device 50 to distinguish between unevenness in thickness of the yarn Y1 and excessive hairiness of the yarn Y1 and detect them. Thus, in the yarn monitoring device 50, the accuracy of hairiness detection can be improved.

The automatic winder 30 includes a machine control device 40a configured to set a measurement length for calculating the standard deviation per predetermined length of yarn Y and output measurement length information on the measurement length to the yarn monitoring device 50. The yarn monitoring device 50 calculates the standard deviation based on the measurement length information output from the machine control device 40a. In this configuration, the standard deviation for each measurement length can be calculated regardless of the speed of yarn Y, and thus the hairiness can be accurately detected.

In the automatic winder 30, after winding of yarn Y1 from a new yarn feeding bobbin SB has been started, when the yarn monitoring device 50 has detected an excessively hairy part at the time the yarn Y1 has been wound for the measurement length, the yarn feeding bobbin SB is considered to be a defective bobbin having much hairiness, the winding of the yarn Y1 is stopped, and the yarn feeding bobbin SB is ejected from the winding unit 32. Thus, when the measurement length is set to be shorter, a defective bobbin can be detected earlier, whereby unnecessary winding of the yarn Y1 can be avoided. On the other hand, when the measurement length is too short, the value of the standard deviation is not stable. In view of this, the measurement length is set to be 1 m or more and 20 m or less, for example.

In the above embodiment, each winding unit 32 preferably includes means for detecting the yarn speed. Furthermore, the corresponding yarn monitoring device 50 preferably includes means for detecting the yarn speed. In this case, the control section of the yarn monitoring device 50 can perform sampling corresponding to the sampling interval length based on the detected yarn speed. Herein, the winding unit 32 may include means for detecting the yarn speed separately from the yarn monitoring device 50. The control section may also perform sampling corresponding to the sampling interval length based on the yarn speed (the yarn speed that is a target winding speed input by the operator) set in a setting device such as the machine control device 40a.

In the above embodiment, the hairiness count can be displayed. However, not only the hairiness count, but also a trend in the hairiness count (or the hairiness quantity) corresponding to the yarn speed may be displayed, for example. In other words, changes in the hairiness count (or the hairiness quantity) may be displayed in response to changes in the yarn speed. Alternatively, for example, a trend in the hairiness count (or the hairiness quantity) may be displayed for each yarn feeding bobbin SB. In other words, changes in the hairiness count (or the hairiness quantity) from the start of unwinding to the completion thereof may be displayed for each yarn feeding bobbin SB.

In the above embodiments, an example of a configuration has been described in which the host devices are the machine control devices 5a, 40a. However, the host devices are not limited to the machine control devices 5a, 40a, and may be a device provided separately from the machine control devices 5a, 40a.

In addition to the above embodiments, when the standard deviation or the mean deviation is calculated, another factor may be combined for the calculation. As the other factor, for example, skewness or kurtosis can be used. By combining the other factor in the calculation of the standard deviation or the mean deviation, the accuracy of the hairiness quantity estimation can be increased.

**Reference Signs List**

1 ... air-jet spinning machine (yarn winder)
3a ... yarn joining device (yarn joining section)
5a ... machine control device (detection section)
5b ... display screen (display section)
7 ... air-jet spinning device (yarn feeding section)
8, 50 ... yarn monitoring device
13 ... winding device (winding section)
20 ... holder
20a, 20b ... wall
22 ... light emitter
24 ... light receiver
26 ... control section (detection section)
30 ... automatic winder (yarn winder)
40a ... machine control device (detection section)
40b ... display screen (display section)
42 ... yarn feeding section
48 ... yarn joining device (yarn joining section)
52 ... winding device (winding section)
R ... traveling area
Y, Y1 ... yarn

**Claims**

1. A yarn monitoring device (8, 50) comprising:

    a light emitter (22) configured to emit light toward a traveling area (R) where yarn (Y) travels;
    a light receiver (24) disposed opposite to the light emitter (22) across the traveling area (R) and configured to receive the light emitted from the light emitter (22) and partially blocked by the yarn (Y) and to output a detection signal corresponding to the amount of the light received; and
    a detection section (5a, 40a) configured to detect, based on the detection signal, a state of the yarn (Y) traveling in the traveling area (R), wherein
    the detection section (5a, 40a) estimates hairiness quantity of the yarn (Y) by extracting a predetermined frequency component from the detection signal and calculating a standard deviation or a mean deviation of the extracted predetermined frequency component, **characterized in that**
    the detection section (5a, 40a) extracts the predetermined frequency component by a frequency filter, wherein a time corresponding to a lower frequency limit of a passband in the frequency filter is set between a time for which the yarn (Y) travels a length of 1 cm and a time for which the yarn (Y) travels a length of 5 cm.

2. The yarn monitoring device (8, 50) according to claim 1, wherein the detection section (5a, 40a) determines whether the hairiness quantity is excessively large, based on a result of comparison between the standard deviation or the mean deviation with a threshold.

3. The yarn monitoring device (8, 50) according to claim 1 or 2, wherein the detection section (5a, 40a) compares the standard deviation or the mean deviation with a threshold and outputs a result of this comparison.

4. The yarn monitoring device (8, 50) according to any one of claims 1 to 3, wherein the detection section (5a, 40a) detects presence of abnormality in appearance of the yarn as the state of the yarn (Y), based on the detection signal.

5. The yarn monitoring device (8, 50) according to any one of claims 1 to 4, wherein the light receiver (24) is a photoelectric conversion element including one pixel.

6. The yarn monitoring device (8, 50) according to any one of claims 1 to 5, comprising a holder (20) configured to hold the light emitter (22) and the light receiver (24) and having the traveling area (R) formed therein, wherein

at least parts, in walls (20a, 20b) of the holder (20) facing the traveling area (R), that hold the light emitter (22) and the light receiver (24) is black in color.

7. The yarn monitoring device (8, 50) according to any one of claims 1 to 6, wherein the detection section (5a, 40a) calculates a hairiness count by substituting the standard deviation or the mean deviation into a conversion formula.

8. The yarn monitoring device (8, 50) according to claim 7, wherein the detection section (5a, 40a) uses the conversion formula corresponding to a type of the yarn (Y) when calculating the hairiness count.

9. The yarn monitoring device (8, 50) according to any one of claims 1 to 7, wherein the detection section (5a, 40a) samples the detection signal at every sampling interval length in the yarn (Y), wherein the sampling interval length is preferably 0.5 mm or more and 5 mm or less.

10. A yarn monitoring method to be performed by a control section in a yarn monitoring device (8, 50), in which a light emitter (22) emits light toward traveling yarn (Y), and a light receiver (24) receives light emitted from the light emitter (22) and partially blocked by the yarn (Y), and outputs a detection signal corresponding to the amount of the light received to the control section, the method comprising:

   an extraction step of extracting a predetermined frequency component from the detection signal; and
   a hairiness quantity estimation step of estimating hairiness quantity of the yarn by calculating a standard deviation or a mean deviation of the predetermined frequency component extracted at the extraction step,

   **characterized in that** the detection section (5a, 40a) extracts the predetermined frequency component by a frequency filter, wherein a time corresponding to a lower frequency limit of a passband in the frequency filter is set between a time for which the yarn (Y) travels a length of 1 cm and a time for which the yarn (Y) travels a length of 5 cm.

11. A yarn winder (1, 30) comprising:

   a yarn feeding section (7) capable of feeding yarn (Y);
   a winding section (13, 52) configured to wind the yarn (Y) fed by the yarn feeding section (7) into a package;
   a yarn joining section (3a, 48) configured to perform yarn joining operation of joining the yarn (Y) fed by the yarn feeding section (7) and the yarn (Y) wound by the winding section (13, 52); and
   the yarn monitoring device (8, 50) according to any one of claims 1 to 9, wherein
   the yarn monitoring device (8, 50) is disposed between the yarn feeding section (7) and the winding section (13, 52).

12. The yarn winder (1, 30) according to claim 11, comprising: a host device configured to set a measurement length for calculating the standard deviation or the mean deviation per predetermined length of the yarn (Y) and output measurement length information on the measurement length to the yarn monitoring device (8, 50), wherein
   the yarn monitoring device (8, 50) calculates the standard deviation or the mean deviation based on the measurement length information output from the host device.

13. A yarn monitoring system comprising:

   the yarn monitoring device (8, 50) according to any one of claims 1 to 9; and
   a host device capable of communicating data to and from the yarn monitoring device (8, 50).

14. The yarn monitoring system according to claim 13, wherein

   the host device transmits measurement length information on the measurement length to the yarn monitoring device (8, 50) for calculating the standard deviation or the mean deviation for each predetermined length of the yarn (Y), and
   the detection section (5a, 40a) calculates the standard deviation or the mean deviation based on the measurement length information received from the host device.

15. The yarn monitoring system according to claim 13 or 14, wherein the host device transmits threshold information on a threshold to the yarn monitoring device (8, 50), and

the yarn monitoring device (8, 50) determines whether the hairiness quantity is excessively large, based on the standard deviation or the mean deviation and the threshold information received from the host device.

16. The yarn monitoring system according to claim 15, wherein

a hairiness count is settable in the host device, and
the host device converts hairiness count information on the hairiness count into the threshold information and transmits the threshold information after this conversion to the yarn monitoring device (8, 50).

17. The yarn monitoring system according to claim 16, wherein

the yarn monitoring device (8, 50) transmits the standard deviation or the mean deviation to the host device, and
the host device receives the standard deviation or the mean deviation transmitted from the yarn monitoring device (8, 50), and calculates the hairiness count by substituting the received standard deviation or the received mean deviation into a conversion formula corresponding to a type of the yarn (Y).

18. The yarn monitoring system according to claim 17, comprising a display section (5b, 40b) configured to display the hairiness count calculated by the host device,

wherein the detection section (5a, 40a) acquires a yarn speed and calculates the standard deviation or the mean deviation for each predetermined length of the yarn (Y), and
the display section (5b, 40b) displays a trend in the hairiness count corresponding to the yarn speed.

**Patentansprüche**

1. Fadenüberwachungsvorrichtung (8, 50), umfassend:

einen Lichtemitter (22), der ausgebildet ist, um Licht in Richtung eines Laufbereichs (R) auszusenden, in dem sich der Faden (Y) bewegt;
einen Lichtempfänger (24), der dem Lichtemitter (22) über den Laufbereich (R) hinweg gegenüberliegend angeordnet und ausgebildet ist, um das vom Lichtemitter (22) ausgesendete und durch den Faden (Y) teilweise abgeschirmte Licht zu empfangen und ein Erfassungssignal auszugeben, das der Menge des empfangenen Lichts entspricht; und
einen Erfassungsabschnitt (5a, 40a), der so ausgebildet ist, um anhand des Erfassungssignals einen Zustand des im Laufbereich (R) laufenden Fadens (Y) zu erfassen, wobei
der Erfassungsabschnitt (5a, 40a) den Haarigkeitsgrad des Fadens (Y) schätzt, indem er eine vorbestimmte Frequenzkomponente aus dem Erfassungssignal extrahiert und eine Standardabweichung oder eine mittlere Abweichung der extrahierten vorbestimmten Frequenzkomponente berechnet, **dadurch gekennzeichnet, dass** der Erfassungsabschnitt (5a, 40a) die vorbestimmte Frequenzkomponente mittels eines Frequenzfilters extrahiert, wobei eine Zeit, die der unteren Frequenzgrenze eines Durchlassbereichs im Frequenzfilter entspricht, zwischen der Zeit, in der der Faden (Y) eine Strecke von 1 cm zurücklegt, und der Zeit, in der der Faden (Y) eine Strecke von 5 cm zurücklegt, festgelegt wird.

2. Fadenüberwachungsvorrichtung (8, 50) nach Anspruch 1, wobei der Erfassungsabschnitt (5a, 40a) auf der Grundlage eines Vergleichsergebnisses zwischen der Standardabweichung oder der mittleren Abweichung und einem Schwellenwert ermittelt, ob der Haarigkeitsgrad übermäßig groß ist.

3. Fadenüberwachungsvorrichtung (8, 50) nach Anspruch 1 oder 2, wobei der Erfassungsabschnitt (5a, 40a) die Standardabweichung oder die mittlere Abweichung mit einem Schwellenwert vergleicht und ein Ergebnis dieses Vergleichs ausgibt.

4. Fadenüberwachungsvorrichtung (8, 50) nach einem der Ansprüche 1 bis 3, wobei der Erfassungsabschnitt (5a, 40a) auf der Grundlage des Erfassungssignals das Vorliegen einer Anomalie im Aussehen des Fadens als Zustand des Fadens (Y) erfasst.

5. Fadenüberwachungsvorrichtung (8, 50) nach einem der Ansprüche 1 bis 4, wobei der Lichtempfänger (24) ein photoelektrisches Wandlerelement ist, das ein Pixel einschließt.

6. Fadenüberwachungsvorrichtung (8, 50) nach einem der Ansprüche 1 bis 5, umfassend einen Halter (20), der so ausgebildet ist, dass er den Lichtemitter (22) und den Lichtempfänger (24) hält, und in dem der Laufbereich (R) ausgebildet ist, wobei

   zumindest Teile der Wände (20a, 20b) des Halters (20), die dem Laufbereich (R) zugewandt sind und den Lichtemitter (22) sowie den Lichtempfänger (24) halten, schwarz sind.

7. Fadenüberwachungsvorrichtung (8, 50) nach einem der Ansprüche 1 bis 6, wobei der Erfassungsabschnitt (5a, 40a) eine Haarigkeitszahl berechnet, indem er die Standardabweichung oder die mittlere Abweichung in eine Umrechnungsformel einsetzt.

8. Fadenüberwachungsvorrichtung (8, 50) nach Anspruch 7, wobei der Erfassungsabschnitt (5a, 40a) bei der Berechnung der Haarigkeitszahl die dem Faden (Y) entsprechende Umrechnungsformel verwendet.

9. Fadenüberwachungsvorrichtung (8, 50) nach einem der Ansprüche 1 bis 7, wobei der Erfassungsabschnitt (5a, 40a) das Erfassungssignal in jedem Abtastintervall im Faden (Y) abtastet, wobei die Länge des Abtastintervalls vorzugsweise 0,5 mm oder mehr und 5 mm oder weniger beträgt.

10. Verfahren zur Fadenüberwachung, das von einer Steuereinheit in einer Fadenüberwachungsvorrichtung (8, 50) durchgeführt wird, bei dem ein Lichtemitter (22) Licht in Richtung des laufenden Fadens (Y) aussendet und ein Lichtempfänger (24) das vom Lichtemitter (22) ausgesendete und vom Faden (Y) teilweise abgeschirmte Licht empfängt und ein der Menge des empfangenen Lichts entsprechendes Erfassungssignal an die Steuereinheit ausgibt, wobei das Verfahren umfasst:

   einen Extraktionsschritt zum Extrahieren einer vorgegebenen Frequenzkomponente aus dem Erfassungssignal; und

   ein Schritt zur Schätzung des Haarigkeitsgrads, bei dem die Haarigkeit des Fadens durch Berechnung der Standardabweichung oder der mittleren Abweichung der im Extraktionsschritt extrahierten vorbestimmten Frequenzkomponente geschätzt wird, **dadurch gekennzeichnet, dass**

   der Erfassungsabschnitt (5a, 40a) die vorgegebene Frequenzkomponente mittels eines Frequenzfilters herausfiltert, wobei eine Zeitspanne, die der unteren Frequenzgrenze eines Durchlassbereichs im Frequenzfilter entspricht, zwischen der Zeit, in der der Faden (Y) eine Strecke von 1 cm zurücklegt, und der Zeit, in der der Faden (Y) eine Strecke von 5 cm zurücklegt, festgelegt wird.

11. Fadenwickler (1, 30) umfassend:

   eine Fadenzuführvorrichtung (7), die in der Lage ist, Faden (Y) zuzuführen;

   einen Aufwickelabschnitt (13, 52), der dazu ausgebildet ist, den von der Fadenzuführvorrichtung (7) zugeführten Faden (Y) zu einer Spule aufzuwickeln;

   einen Fadenverbindungsabschnitt (3a, 48), der ausgebildet ist, um einen Fadenverbindungsvorgang durchzuführen, bei dem der von der Fadenzuführvorrichtung (7) zugeführte Faden (Y) mit dem von dem Aufwickelabschnitt (13, 52) aufgewickelten Faden (Y) verbunden wird; und

   die Fadenüberwachungsvorrichtung (8, 50) nach einem der Ansprüche 1 bis 9, wobei

   die Fadenüberwachungsvorrichtung (8, 50) zwischen der Fadenzuführvorrichtung (7) und dem Aufwickelabschnitt (13, 52) angeordnet ist.

12. Fadenwickler (1, 30) nach Anspruch 11, umfassend: eine Host-Vorrichtung, die ausgebildet ist, um eine Messlänge zur Berechnung der Standardabweichung oder der mittleren Abweichung pro vorgegebene Länge des Fadens (Y) festzulegen und Messlängeninformationen an die Fadenüberwachungsvorrichtung (8, 50) auszugeben, wobei die Fadenüberwachungsvorrichtung (8, 50) die Standardabweichung oder die mittlere Abweichung auf der Grundlage der von der Host-Vorrichtung ausgegebenen Messlängeninformationen berechnet.

13. Fadenüberwachungssystem, umfassend:

   die Fadenüberwachungsvorrichtung (8, 50) nach einem der Ansprüche 1 bis 9; und

   eine Host-Vorrichtung, die Daten mit der Fadenüberwachungsvorrichtung (8, 50) austauschen kann.

14. Fadenüberwachungssystem nach Anspruch 13, wobei

die Host-Vorrichtung Messlängeninformationen an die Fadenüberwachungsvorrichtung (8, 50) übermittelt, um die Standardabweichung oder die mittlere Abweichung für jede vorgegebene Länge des Fadens (Y) zu berechnen, und
der Erfassungsabschnitt (5a, 40a) die Standardabweichung oder die mittlere Abweichung auf der Grundlage der von der Host-Vorrichtung empfangenen Messlängeninformationen berechnet.

15. Fadenüberwachungssystem nach einem der Ansprüche 13 oder 14, wobei die Host-Vorrichtung Schwellenwertinformationen über einen Schwellenwert an die Fadenüberwachungsvorrichtung (8, 50) überträgt, und
die Fadenüberwachungsvorrichtung (8, 50) anhand der Standardabweichung oder der mittleren Abweichung sowie der von der Hostvorrichtung empfangenen Schwellenwertinformationen ermittelt, ob der Haarigkeitsgrad übermäßig hoch ist.

16. Fadenüberwachungssystem nach Anspruch 15, wobei

in der Host-Vorrichtung eine Haarigkeitszahl einstellbar ist, und
die Host-Vorrichtung die Haarigkeitszahl-Informationen der Haarigkeitszahl in Schwellenwertinformationen umwandelt und diese Schwellenwertinformationen nach der Umwandlung an die Fadenüberwachungsvorrichtung (8, 50) überträgt.

17. Fadenüberwachungssystem nach Anspruch 16, wobei

die Fadenüberwachungsvorrichtung (8, 50) die Standardabweichung oder die mittlere Abweichung an die Host-Vorrichtung übermittelt, und
die Host-Vorrichtung die von der Fadenüberwachungsvorrichtung (8, 50) übermittelte Standardabweichung oder mittlere Abweichung empfängt und die Haarigkeitszahl berechnet, indem es die empfangene Standardabweichung oder mittlere Abweichung in eine Umrechnungsformel einsetzt, die dem Typ des Fadens (Y) entspricht.

18. Fadenüberwachungssystem nach Anspruch 17, umfassend einen Anzeigeabschnitt (5b, 40b), der dazu ausgelegt ist, die von der Host-Vorrichtung berechnete Haarigkeitszahl anzuzeigen,

wobei der Erfassungsabschnitt (5a, 40a) eine Fadengeschwindigkeit erfasst und die Standardabweichung oder die mittlere Abweichung für jede vorgegebene Länge des Fadens (Y) berechnet, und
der Anzeigeabschnitt (5b, 40b) einen Trend der Haarigkeitszahl anzeigt, der der Fadengeschwindigkeit entspricht.

**Revendications**

1. Dispositif de surveillance de fil (8, 50) comprenant :

une partie d'émission de lumière (22) configurée pour émettre de la lumière en direction d'une zone de déplacement (R) dans laquelle le fil (Y) se déplace ;
une partie de réception de lumière (24) disposée à l'opposé de la partie d'émission de lumière (22) à travers la zone de déplacement (R) et configurée pour recevoir la lumière émise depuis la partie d'émission de lumière (22) et partiellement bloquée par le fil (Y) et pour délivrer en sortie un signal de détection correspondant à la quantité de la lumière reçue ; et
une section de détection (5a, 40a) configurée pour détecter, sur la base du signal de détection, un état du fil (Y) se déplaçant dans la zone de déplacement (R), dans lequel
la section de détection (5a, 40a) estime la quantité de pilosité du fil (Y) en extrayant un composant de fréquence prédéterminé à partir du signal de détection et en calculant un écart-type ou un écart moyen du composant de fréquence prédéterminé extrait, **caractérisé en ce que** la section de détection (5a, 40a) extrait le composant de fréquence prédéterminé au moyen d'un filtre de fréquence, dans lequel un temps correspondant à une limite de fréquence inférieure d'une bande passante dans le filtre de fréquence est défini entre un temps pendant lequel le fil (Y) se déplace sur une longueur de 1 cm et un temps pendant lequel le fil (Y) se déplace sur une longueur de 5 cm.

2. Dispositif de surveillance de fil (8, 50) selon la revendication 1, dans lequel la section de détection (5a, 40a) détermine si la quantité de pilosité du fil est excessivement grande, sur la base d'un résultat de comparaison entre l'écart-type ou l'écart moyen et un seuil.

3. Dispositif de surveillance de fil (8, 50) selon la revendication 1 ou la revendication 2, dans lequel la section de détection (5a, 40a) compare l'écart-type ou l'écart moyen à un seuil et délivre en sortie un résultat de cette comparaison.

4. Dispositif de surveillance de fil (8, 50) selon l'une quelconque des revendications 1 à 3, dans lequel la section de détection (5a, 40a) détecte la présence d'une anomalie dans l'aspect du fil en tant qu'état du fil (Y), sur la base du signal de détection.

5. Dispositif de surveillance de fil (8, 50) selon l'une quelconque des revendications 1 à 4, dans lequel la partie de réception de lumière (24) est un élément de conversion photoélectrique incluant un pixel.

6. Dispositif de surveillance de fil (8, 50) selon l'une quelconque des revendications 1 à 5, comprenant un support (20) configuré pour maintenir la partie d'émission de lumière (22) et la partie de réception de lumière (24) et présentant la zone de déplacement (R) formée en son sein, dans lequel
au moins des parties, dans des parois (20a, 20b) du support (20) faisant face à la zone de déplacement (R), qui maintiennent la partie d'émission de lumière (22) et la partie de réception de lumière (24), sont de couleur noire.

7. Dispositif de surveillance de fil (8, 50) selon l'une quelconque des revendications 1 à 6, dans lequel la section de détection (5a, 40a) calcule un indice de pilosité en substituant l'écart-type ou l'écart moyen dans une formule de conversion.

8. Dispositif de surveillance de fil (8, 50) selon la revendication 7, dans lequel la section de détection (5a, 40a) utilise la formule de conversion correspondant à un type du fil (Y) lors du calcul de l'indice de pilosité.

9. Dispositif de surveillance de fil (8, 50) selon l'une quelconque des revendications 1 à 7, dans lequel la section de détection (5a, 40a) échantillonne le signal de détection à chaque longueur d'intervalle d'échantillonnage dans le fil (Y), dans lequel la longueur d'intervalle d'échantillonnage est de préférence de 0,5 mm ou plus et de 5 mm ou moins.

10. Procédé de surveillance de fil devant être réalisé par une section de commande dans un dispositif de surveillance de fil (8, 50), dans lequel une partie d'émission de lumière (22) émet de la lumière en direction d'un fil (Y) en déplacement, et une partie de réception de lumière (24) reçoit la lumière émise depuis la partie d'émission de lumière (22) et partiellement bloquée par le fil (Y), et délivre en sortie un signal de détection correspondant à la quantité de la lumière reçue à la section de commande, le procédé comprenant :

une étape d'extraction consistant à extraire un composant de fréquence prédéterminé du signal de détection ; et
une étape d'estimation de quantité de pilosité consistant à estimer la quantité de pilosité du fil en calculant un écart-type ou un écart moyen du composant de fréquence prédéterminé extrait à l'étape d'extraction,
**caractérisé en ce que**
la section de détection (5a, 40a) extrait le composant de fréquence prédéterminé au moyen d'un filtre de fréquence, dans lequel un temps correspondant à une limite de fréquence inférieure d'une bande passante dans le filtre de fréquence est défini entre un temps pendant lequel le fil (Y) se déplace sur une longueur de 1 cm et un temps pendant lequel le fil (Y) se déplace sur une longueur de 5 cm.

11. Enrouleur de fil (1, 30) comprenant :

une section d'alimentation en fil (7) apte à alimenter un fil (Y) ;
une section d'enroulement (13, 52) configurée pour enrouler le fil (Y) alimenté par la section d'alimentation en fil (7) en un paquet ;
une section de raccordement de fil (3a, 48) configurée pour réaliser une opération de raccordement de fil consistant à raccorder le fil (Y) alimenté par la section d'alimentation en fil (7) et le fil (Y) enroulé par la section d'enroulement (13, 52) ; et
le dispositif de surveillance de fil (8, 50) selon l'une quelconque des revendications 1 à 9, dans lequel
le dispositif de surveillance de fil (8, 50) est disposé entre la section d'alimentation en fil (7) et la section d'enroulement (13, 52).

12. Enrouleur de fil (1, 30) selon la revendication 11, comprenant : un dispositif hôte configuré pour définir une longueur de mesure permettant de calculer l'écart-type ou l'écart moyen par longueur prédéterminée du fil (Y) et délivrer en sortie des informations de longueur de mesure sur la longueur de mesure au dispositif de surveillance de fil (8, 50), dans lequel

le dispositif de surveillance de fil (8, 50) calcule l'écart-type ou l'écart moyen sur la base des informations de longueur de mesure délivrées en sortie par le dispositif hôte.

13. Système de surveillance de fil comprenant :

le dispositif de surveillance de fil (8, 50) selon l'une quelconque des revendications 1 à 9 ; et
un dispositif hôte apte à communiquer des données vers et depuis le dispositif de surveillance de fil (8, 50).

14. Système de surveillance de fil selon la revendication 13, dans lequel

le dispositif hôte transmet des informations de longueur de mesure sur la longueur de mesure au dispositif de surveillance de fil (8, 50) pour calculer l'écart-type ou l'écart moyen pour chaque longueur prédéterminée du fil (Y), et
la section de détection (5a, 40a) calcule l'écart-type ou l'écart moyen sur la base des informations de longueur de mesure reçues du dispositif hôte.

15. Système de surveillance de fil selon la revendication 13 ou la revendication 14, dans lequel le dispositif hôte transmet des informations de seuil sur un seuil au dispositif de surveillance de fil (8, 50), et
le dispositif de surveillance de fil (8, 50) détermine si la quantité de pilosité du fil est excessivement grande, sur la base de l'écart-type ou de l'écart moyen et des informations de seuil reçues du dispositif hôte.

16. Système de surveillance de fil selon la revendication 15, dans lequel un indice de pilosité peut être défini dans le dispositif hôte, et
le dispositif hôte convertit des informations d'indice de pilosité en informations de seuil et transmet les informations de seuil après cette conversion au dispositif de surveillance de fil (8, 50).

17. Système de surveillance de fil selon la revendication 16, dans lequel

le dispositif de surveillance de fil (8, 50) transmet l'écart-type ou l'écart moyen au dispositif hôte, et
le dispositif hôte reçoit l'écart-type ou l'écart moyen transmis depuis le dispositif de surveillance de fil (8, 50), et calcule l'indice de pilosité en substituant l'écart-type reçu ou l'écart moyen reçu dans une formule de conversion correspondant à un type du fil (Y).

18. Système de surveillance de fil selon la revendication 17, comprenant une section d'affichage (5b, 40b) configurée pour afficher l'indice de pilosité calculé par le dispositif hôte,

dans lequel la section de détection (5a, 40a) acquiert une vitesse du fil et calcule l'écart-type ou l'écart moyen pour chaque longueur prédéterminée du fil (Y), et
la section d'affichage (5b, 40b) présente une tendance de l'indice de pilosité correspondant à la vitesse du fil.

*Fig.1*

## Fig.2

*Fig.3*

# Fig.4

EP 4 130 362 B1

**Fig.5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005299037 A

- JP H02257382 A

**Non-patent literature cited in the description**

- A comparison of mass parameters determination using capacitive and optical sensors. **CARVALHO V et al.** PROCEDIA CHEMISTRY. ELSEVIER, 01 September 2009, vol. 1, 766-769